**Europäisches Patentamt**

**European Patent Office**

**Office européen des brevets**

(11) Veröffentlichungsnummer: **0 003 948**
**A1**

## EUROPÄISCHE PATENTANMELDUNG

(21) Anmeldenummer: **78100401.5**

(22) Anmeldetag: **14.07.78**

(51) Int. Cl.²: **A 61 N 1/04**

(30) Priorität: **19.07.77 DE 2732547**

(43) Veröffentlichungstag der Anmeldung:
**19.09.79 Patentblatt 79/19**

(84) Benannte Vertragsstaaten:
**BE CH DE FR GB LU NL SE**

(71) Anmelder: **Bisping, Hans-Jürgen, Dipl.-Ing.**
**Tittardshang 12**
**D-5100 Aachen-Laurensberg(DE)**

(72) Erfinder: **Bisping, Hans-Jürgen, Dipl.-Ing.**
**Tittardshang 12**
**D-5100 Aachen-Laurensberg(DE)**

(74) Vertreter: **Christiansen, Henning, Dipl.-Ing.**
**Basseler Strasse 172**
**D-1000 Berlin 45(DE)**

(54) **Implantierbare Elektrode.**

(57) Implantierbare Elektrode, insbesondere zum Implantieren in einer Herzkammer zur Stimulation des Herzmuskels, mit einer isolierten Elektrodenzuleitung (2) und mindestens einem Befestigungselement (5) zum Fixieren des Elektrodenendes, um einen die Stimulationsfläche bildenden leitfähigen Bereich desselben mit dem Körpergewebe in Kontakt zu bringen, bei der das Befestigungselement im Bereich des den Elektrodenkopf bildenden Elektrodenendes (3,11) in Axialrichtung der Elektrodenzuleitung in Bezug auf dessen Frontfläche zurückversetzt, für die Einführungsphase im wesentlichen innerhalb der vom Körpergewebe tangierten Aussenkontur des Elektrodenkopfes unterbringbar und zum Fixieren des Elektrodenendes derart relativ zu der Aussenkontur bewegbar angeordnet ist, dass es zum Eingriff in die Geweboberfläche in Bezug auf die Einführungsrichtung der Elektrode hinter der Frontfläche des Elektrodenkopfes über die Aussenkontur hinaus gelangt.

EP 0 003 948 A1

./.

Beschreibung

Die Erfindung betrifft eine implantierbare Elektrode, insbesondere zum Implantieren in einer Herzkammer zur Stimulation des Herzmuskels, mit einer isolierten Elektrodenzuleitung und mindestens einem Befestigungselement zum Fixieren des Elektrodenendes, um einen die Stimulationsfläche bildenden leitfähigen Bereich desselben mit dem Körpergewebe in Kontakt zu bringen.

Implantierbare Elektroden stellen die elektrisch leitende Verbindung zwischen einem Impulsgenerator und dem zu stimulierenden Gewebe im Körper des Patienten dar. Elektroden von Herzschrittmachern dienen dabei zum Übertragen von elektrischen Signalen zwischen dem in den Körper des Patienten implantierten künstlichen Herzschrittmacher und dem im Herzen befindlichen Ende der Elektrode. Da der künstliche Herzschrittmacher selbst aus operativen Gründen leicht zugänglich in der Nähe der Körperoberfläche angeordnet sein muß, die Erfassung von Herzsignalen bzw. die Abgabe von Stimulationsimpulsen aber direkt am Herzmuskel erfolgen, wird durch die Elektrode die entsprechende Entfernung im Körper überbrückt. Bei endokardialer Implantationsweise wird die Elektrode durch eine Vene ins Herz vorgeschoben. Schwierigkeiten ergeben sich dabei durch Dislokationen von nicht ausreichend fixierbaren Elektro-

/2

denköpfen infolge der Bewegungen des Herzens oder des Blutstroms. Vor allen Dingen, wenn die aus medizinischer Sicht häufig wünschenswerte Fixierung im Vorhof vorgenommen werden soll, ist die Dislokationsneigung wegen der glatten Vorhofmuskulatur sehr groß.

Es sind Elektroden bekannt, bei denen mechanische Fixierungshilfen in Form von Metall- oder Kunststoffhaken eine feste Verankerung im Herzen bewirken sollen. Bei einer aus der DE-OS 20 53 919 bekannten Elektrode, die einen Draht-Widerhaken an ihrem Kopf trägt, ist die Wahl des Stimulationsortes im Herzen durch die Steifigkeit eines Führungskatheters erschwert, der die Aufgabe hat, beim Einführen der Elektrode die Draht-Widerhaken vom Körpergewebe fernzuhalten.

Bei anderen Hakensystemen wird der Druck eines im Innern der Elektrode liegenden Führungsdrahtes (Mandrin) dazu verwendet, einen Hakenmechanismus, der während der Einführungsphase zurückgezogen ist, auszufahren und in das Herzgewebe vorzuschieben (DE-PS 2 133 304 und DE-PS 2 149 449). Dabei treten aus der Stirnseite des Elektrodenkopfes schräg nach vorn gerichtet Stahl- oder Kunststoffhäkchen aus, die jedoch nur dann ein zuverlässiges Verhaken der Elektrode ermöglichen, wenn der Elektrodenkopf senkrecht auf der Gewebeoberfläche steht. Kommt der Elektrodenkopf unter einem spitzen Winkel in Bezug auf die Gewebeoberfläche zu liegen, so kann es vorkommen, daß nur eines der Häkchen mit dem Gewebe in Eingriff kommt. Durch die Konstruktion der bekannten Elektrode ist es bedingt, daß bei Eingreifen nur eines Häkchens kein sicherer Sitz gewährleistet ist und bei entsprechend gerichteten, etwa durch den Blutstrom oder Herzbewegungen hervorgeru-

/3

fenen Kräften eine Dislokation der Elektrode bewirkt wird.
Besonders nachteilig ist dabei, daß sich die schräg nach
vorn gerichteten Häkchen im Laufe der Zeit durch das Herzgewebe bohren und zu gefährlichen Perforationen führen
können.

Weiterhin ist es aus der DE-OS 26 13 044 bekannt, am Ende
der Elektrodenzuleitung eine spiralförmige Wendel zu
befestigen, die an der gewünschten Stelle in das Herzgewebe durch Drehen der Elektrodenzuleitung eingeschraubt
wird. Diese bekannte Anordnung hat bestimmungsgemäß eine
Stichverletzung im Herzmuskelgewebe zur Folge, so daß es
zu einer Traumatisierung und fibrotischen Gewebereaktion
kommen kann. Da über die Wendel auch die elektrische Stimulation erfolgt, können die sich zwangsläufig ergebende
Gewebereaktionen zu einem Ansteigen der Reizschwelle des
Herzens führen. Wenn die Reizschwelle die Amplitude der
vom Herzschrittmacher abgegebenen Impulse überschreitet,
ist der künstliche Herzschrittmacher wirkungslos.

Der Erfindung liegt die Aufgabe zugrunde, die genannten
Nachteile zu vermeiden und eine Elektrode zu schaffen, die
zum einen leicht und sicher einführbar sowie dislokationssicher ist und zum anderen eine gute elektrische Stimulation des Herzmuskels bei im wesentlichen gleichbleibender
Reizschwelle ermöglicht.

Diese Aufgabe wird bei einer Elektrode der oben angegebenen Gattung dadurch gelöst, daß das Befestigungselement im
Bereich des den Elektrodenkopf bildenden Elektrodenendes
in Axialrichtung der Elektrodenzuleitung in Bezug auf dessen Frontfläche zurückversetzt, für die Einführungsphase
im wesentlichen innerhalb der vom Körpergewebe tangierten

0003948

Außenkontur des Elektrodenkopfes unterbringbar und zum Fixieren des Elektrodenendes derart relativ zu der Außenkontur bewegbar angeordnet ist, daß es zum Eingriff in die Gewebeoberfläche in Bezug auf die Einführungsrichtung der Elektrode hinter der Frontfläche des Elektrodenkopfes über die Außenkontur hinaus gelangt.

Durch diese Ausgestaltung der Elektrode ist es in vorteilhafter Weise möglich, daß die Stimulation des betreffenden Körperorgans im wesentlichen über die bestimmungsgemäße Stimulationsfläche erfolgt, während das Befestigungselement, entsprechend seiner eigentlichen Funktion, den Elektrodenkopf in der gewünschten Lage festhält. Der Erfindung liegt dabei die Erkenntnis zugrunde, daß der Elektrodenkopf in den wenigsten Fällen beim Einführen der Elektrode senkrecht auf das zu stimulierende Gewebe auftrifft. Durch das im Herzen liegende Trabekelwerk wird sich beim Kontakt des Elektrodenkopfes mit der Gewebeoberfläche immer Gewebe finden, welches auch den zurückliegenden Teil des Elektrodenkopfes berührt bzw. welches mit dem Elektrodenkopf einen mehr oder weniger spitzen Winkel bildet. Durch ein hinter der Frontfläche des Elektrodenkopfs austretendes Befestigungselement wird dieser Umstand ausgenutzt, um in dieser relativ stabilen Lage des Elektrodenendes eine sichere Fixierung der Elektrode zu erreichen.

In der fixierten Position liegt das Elektrodenende mit mäßigem Druck an der Gewebeoberfläche an, so daß ohne störende Fibrinbildung eine Stimulation mit geringem Potential möglich ist.

/5

Bevorzugterweise ist das Befestigungselement in im wesentlichen radialer Richtung in bezug auf die Achse der Elektrodenzuleitung über die Außenkontur hinaus bewegbar angeordnet.

In weiterer vorteilhafter Ausgestaltung der Erfindung enthält das Befestigungselement mindestens einen spiralförmigen Fixierhaken, der mindestens etwa eine Federwindung aufweist und am Elektrodenkopf befestigt und mittels einer Sperrvorrichtung innerhalb der Außenkontur gehalten ist. Der Fixierhaken kann in einer vorzugsweise exzentrisch angeordneten Ringnut des Elektrodenkopfes gelagert sein. Anstelle eines Fixierhakens können auch andere den Elektrodenkopf im Gewebe festhaltende Elemente, wie beispielsweise Klammern etc. Verwendung finden. Gemäß einer anderen bevorzugten Ausführung der Erfindung kann der spiralförmige Fixierhaken starr ausgeführt, im Elektrodenkopf bzw. am Ende der Elektrodenleitung drehbar, exzentrisch gelagert und nach der Einführungsphase nach außerhalb der Außenkontur des Elektrodenkopfes schwenkbar sein.

Gleich in welcher Weise der Fixierhaken ausgestaltet ist, während der Einführungsphase befindet er sich innerhalb der Außenkontur des Elektrodenkopfes, so daß die Elektrode in eine Vene eingebracht und durch diese hindurch zu einer gewünschten Stelle und Lage im Herzen geschoben werden kann, ohne daß die Vene oder Klappen verletzt werden. Wenn die gewünschte Reizstelle in gewohnter Weise gefunden ist, wird die Sperrvorrichtung des federnden Fixierhakens gelöst oder der starre Fixierhaken ausgeschwenkt, so daß dieser aus der Außenkontur des Elektrodenkopfes hinausragt. Durch Aufbringen eines Drehmomentes auf die Elektro-

0003948

denzuleitung, vorzugsweise an dem dem Elektrodenkopf gegenüberliegenden Ende, kommt der scharfe Teil der Fixierhaken mit dem Herzgewebe in Eingriff; der Drehwinkel ist im wesentlichen durch die geometrischen Abmessungen des Fixierhakens bzw. der Ringnut begrenzt.

Der Fixierhaken kann aus körperverträglichem Metall in beliebiger Querschnittsform hergestellt und gegenüber dem Elektrodenkopf bzw. der Elektrodenzuleitung isoliert oder auch mit dieser leitend verbunden sein. Der Fixierhaken kann auch aus elastischem Kunststoff hergestellt sein. Durch eine Drehbewegung in entgegengesetzter Richtung ist die Fixierung jederzeit wieder lösbar. Wenn der Fixierhaken eine schraubenförmige Steigung aufweist, wird durch die Drehbewegung einerseits ein axialer Vorschub erzielt und andererseits wird beim Zurückziehen der Elektrode ein Verhaken im Gewebe bzw. in der Venenintima verhindert. Die Gefahr eines selbständigen Herausdrehens des Fixierhakens ist verringert, wenn der Fixierhaken und/oder der Elektrodenkopf im Bereich seiner Außenkontur Widerhaken aufweisen.

Die Elektrode gemäß der Erfindung kann beispielsweise eine als Sperrfaden ausgebildete freigebbare Sperrvorrichtung aufweisen, wobei der Sperrfaden entlang der Elektrodenzuleitung, und zwar innerhalb oder außerhalb derselben, geführt und im Bereich der Fixierhaken zumindest auf einer Seite radial gehalten sein kann. Der Sperrfaden kann dazu in einer in Axialrichtung im Elektrodenkopf verlaufenden Nut oder Bohrung geführt und/oder mittels eines den Elektrodenkopf umschließenden Fadens befestigt sein.

Die Sperrvorrichtung kann auch einen Sperrhaken aufweisen, der innerhalb des Elektrodenkopfes gelagert ist und durch Verschieben den Fixierhaken freigibt. Gemäß einer weiteren Ausgestaltung der Sperrvorrichtung ist ein radial gehaltener Schmelzdraht vorgesehen, bei dem mindestens ein Pol seiner mit einer Stromquelle verbindbaren Zuleitungen gegenüber dem Potential des Elektrodenkopfes, d.h. dem der Zuleitung für die Übertragung der Stimulationsimpulse, elektrisch isoliert ist. Durch das Schmelzen des Drahtes wird der Fixierhaken frei. Wird je nach Ausgestaltung der Elektrodenzuleitung ein Führungsrohr verwendet, so kann dieses während der Einführphase den oder die Fixierhaken umschließen.

Bei Ausgestaltung des Elektrodenkopfes ist es vorteilhaft, wenn die Stimulationsfläche gegenüber dem restlichen Kopf isoliert und im Bereich der Frontfläche des Elektrodenkopfes angeordnet ist.

Gemäß einer anderen Ausgestaltung ist die Stimulationsfläche bis in den Bereich der Außenkontur des Elektrodenkopfes geführt, der dem über die Außenkontur hinausragenden Teil des Fixierhakens benachbart ist. Dadurch ist sichergestellt, daß ein großer Teil der mit dem Gewebe in Kontakt kommenden Oberfläche des Elektrodenkopfes von der Stimulationsfläche eingenommen wird, die damit mit der Gewebeoberfläche in wirksamer elektrischer Verbindung steht.

Das Auslösen des Befestigungselements kann - gegebenenfalls durch Freigabe einer geeigneten Sperrvorrichtung - bei über die Vene in das Herzinnere eingeführtem Elektrodenkopf entweder direkt über ein Betätigungselement oder

/8

auch mittelbar über ein zwischengeschaltetes weiteres Element von außen her vorgenommen werden. Das letztgenannte Element dichtet bei einigen Ausführungsformen der Erfindung gleichzeitig in vorteilhafter Weise das Innere der Elektrodenzuführung gegen eindringende Körperflüssigkeit ab.

Weitere bevorzugte Ausführungsformen der Erfindung sind in den Unteransprüchen angegeben bzw. in den Zeichnungen vergrößert dargestellt und werden nachfolgend einschließlich ihrer Vorteile näher beschrieben. Es zeigen:

Fig. 1     eine schematische Teildarstellung einer ersten Ausführungsform der erfindungsgemäßen Elektrode im Längsschnitt durch den Elektrodenkopf mit einem elastischen Fixierhaken, der durch einen Sperrfaden gehalten ist,

Fign. 2 und 3     einen Schnitt durch den Elektrodenkopf gemäß Fig. 1 entlang den Linien II - II in gesperrter bzw. entriegelter Stellung des Fixierhakens,

Fig. 4     einen Elektrodenkopf gemäß Fig. 1, bei dem die Sperrvorrichtung als Schmelzdraht ausgebildet ist,

Fig. 5     einen Längsschnitt durch eine weitere Ausführungsform der Elektrode gemäß Fig. 1 im Bereich des Elektrodenkopfes bei dem die Stimulationsfläche die Frontfläche des Elektrodenkopfes bildet und der Fixierhaken isoliert befestigt ist und eine schraubenförmige Steigung aufweist,

Fig. 6     einen Längsschnitt durch eine Variante des Elektrodenkopfes gemäß Fig. 5 mit seitlicher Stimulationsfläche,

Fig. 7     einen Längsschnitt durch eine weitere Ausführungsform der Elektrode im Bereich des Elektrodenkopfes, bei der der Fixierhaken über ein zusätzliches Element betätigt wird,

Fig. 8     eine Detaildarstellung einer Variante der Ausführungsform gemäß Fig. 7,

Fig. 9     eine Variante des Fixierhakens (der Elektrodenkopf ist im Querschnitt dargestellt) für die Ausführungsform gemäß Fig. 7 sowie

Fig. 10     einen Längsschnitt durch einen Teil des Elektrodenkopfes entlang der Linie X-X in Fig. 9.

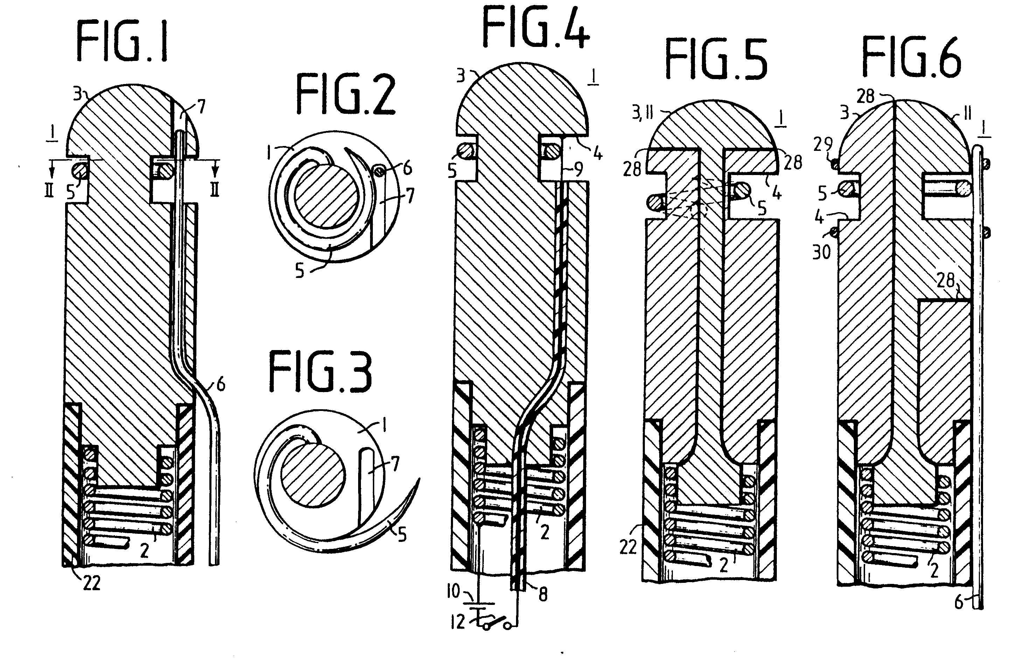

In den Figuren sind verschiedene Ausführungsformen - und Varianten dieser Ausführungsformen - der erfindungsgemäßen Elektrode dargestellt. In den Fign. 1 und 4 bis 8 ist jeweils das einen Elektrodenkopf 1 bildende herznahe Ende der betreffenden Ausführung schematisch im Längsschnitt wiedergegeben. Der Elektrodenkopf wird beim Einführen der Elektrode durch die Vene vorangeführt und weist dabei eine Form auf, bei der keines der Befestigungsteile wesentlich über seine Außenkontur ragen und so den Einführungsvorgang behindern kann. Wenn er die vorgesehene Position in der Herzkammer bzw. im Herzvorhof erreicht hat, so kann - wie es weiter unten noch näher erläutert werden wird - zunächst eine Potentialmessung vorgenommen werden, bevor das Befestigungselement zum Verhaken des Elektrodenkopfes ausgefahren wird. Das Ausfahren des Befestigungselements zum Verhaken in der Herzkammer wird durch den Arzt vom entfernten Ende der Elektrodenzuleitung her ausgelöst, wenn er der Meinung ist, daß der Elektrodenkopf eine gün-

/10

0003948

stige Lage erreicht hat. Die Ausführung der Zuleitung der Elektrode einschließlich der Mittel zum Verbinden der Elektrode mit dem eigentlichen Herzschrittmacher entspricht den derzeit bekannten Ausführungen, so daß auf eine nähere Darstellung verzichtet werden konnte. Als Werkstoffe für die nachfolgenden Ausführungsbeispiele werden körperverträgliche Materialien verwendet, wie sie dem Fachmann aus der einschlägigen Literatur bekannt sind und entsprechend den gewünschten Eigenschaften (Metall oder Isolator) ausgewählt werden können.

In Fig. 1 ist ein Elektrodenkopf 1 wiedergegeben, der an eine gewendelte Elektrodenzuleitung 2 angeschlossen ist, welche von einer schlauchförmigen elastischen Isolierung 22 umgeben ist. Den vorderen Abschluß des Elektrodenkopfes bildet eine Frontfläche 3, welche sphärisch ausgebildet ist. Gegenüber dieser Frontfläche zurückversetzt ist das Befestigungselement (Fixierhaken 5) angeordnet, das im eingefahrenen Zustand in eine umlaufende Ringnut 4 eingelassen ist. Damit ist die Frontfläche 3 des Elektrodenkopfes völlig frei von Befestigungsmitteln und kann eine Form aufweisen, welche einerseits den Einführungsvorgang möglichst wenig behindert und andererseits einen ausgedehnten Kontaktbereich umfaßt, der durch großflächige Berührung mit der Gewebeoberfläche eine gute Stimulation ermöglicht.

Die das Befestigungselement aufnehmende, eine Ausnehmung bildende Ringnut 4 ist so bemessen, daß der sich mit der Gewebeoberfläche verhakende Teil des Befestigungselements soweit innerhalb der Außenkontur verschwinden kann, daß er beim Einführungsvorgang nicht mit dem am Elektrodenkopf anliegenden Gewebe in Eingriff kommt, auch wenn sich dieses an den Elektrodenkopf anschmiegt. Die Ausnehmung ist dabei mehr oder weniger weit gegenüber der Frontfläche 3 des Elektrodenkopfes 1 zurückversetzt.

Die genaue geometrische Bemessung hängt vom vorgesehenen Anwendungsgebiet ab, muß aber derart sein, daß, wenn der Elektrodenkopf 1 beim Einführen auf das zu stimulierende Gewebe auftrifft und dabei - sich auf der Gewebeoberfläche abstützend - einen mehr oder weniger spitzen Winkel zur Gewebeoberfläche einnimmt, das Befestigungselement (bzw. wenn mehrere vorgesehen sind, mindestens eines derselben) im ausgefahrenen Zustand ebenfalls die Gewebeoberfläche sicher erreicht und mit dieser in Eingriff kommt, wenn die Elektrode anschließend um ihre Achse gedreht wird. Der an die Ringnut 4 anschließende, den Übergang zur Elektrodenzuleitung 2 bildende Schaft ist in den wiedergegebenen schematischen Darstellungen zur Erhöhung der Übersichtlichkeit relativ lang ausgeführt und kann - je nach den gestellten Anforderungen - auch kürzer sein.

Die Form und Anbringung des in Fig. 1 dargestellten, das Befestigungselement bildenden spiralförmigen Fixierhakens 5 geht aus den Fign. 2 und 3 hervor. Er ist im wesentlichen spiralförmig ausgebildet und wird durch eine freigebbare Sperrvorrichtung, welche durch einen Sperrfaden 6 gebildet wird, im wesentlichen innerhalb der Außenkontur der Elektrode gehalten. Der Sperrfaden 6 ist dabei lose in eine einen Schlitz bildende Nut 7 eingefügt und stützt sich auf deren Grund ab. In eine derartige Nut läßt sich der Sperrfaden 6 bei der Montage leicht einlegen. Der Fixierhaken 5 beteht aus einem Federwerkstoff und ragt in seiner entspannten Stellung über die Außenkontur der Elektrode hinaus.

Aus räumlichen Gründen ist es günstig, wenn die den Fixierhaken 5 aufnehmende Ringnut 4 exzentrisch ausgebildet ist, wie es ebenfalls aus den Fign. 2 und 3 hervorgeht. Zum Einführen der Elektrode bildet der Sperrfaden 4 eine Schranke, welche das unter Spannung stehende freie Ende des Fixierhakens nicht überwinden kann (Fig. 2). Nachdem der Elektrodenkopf seine vorgesehene Position erreicht hat, genügt es, am Sperrfaden 6, der zunächst innerhalb des Elektrodenkopfes und dann außerhalb, parallel zur Elektrodenzuleitung 2 verläuft und dessen anderes Ende für den Arzt zugänglich ist, zu ziehen, um den Fixierhaken 5 zu befreien und in die ausgefahrene Position gelangen zu lassen (Fig. 3). Durch eine entsprechende Drehung des Elektrodenkopfes, die durch Tordieren des entfernten Endes der Zuleitung bewirkt werden kann, gelangt er mit dem Gewebe in Eingriff.

Statt in eine schlitzförmige Nut kann der Sperrfaden auch in eine Bohrung 7 eingeführt sein, die im Endbereich des Elektrodenkopfes 1 in einer Sackbohrung oder einer Durchgangsbohrung endet. Hierbei ist der Faden sehr stabil gelagert und gegen Herausgleiten besonders geschützt.

Der Sperrfaden 6 kann statt, wie bei dem in Fig. 1 dargestellten Ausführungsbeispiel nach außerhalb des Elektrodenkopfes zu gelangen, durch einen entsprechend ausgebildeten Führungskanal auch in das Innere der Elektrodenzuleitung 2 hinein und durch diese hindurch geführt werden. Das bietet den Vorteil, daß der Faden sich nicht durch beim Einführen entstehende Reibungskräfte zurückziehen und den Fixierhaken vorzeitig freigeben kann.

/13

0003948

Bei der in Fig. 4 dargestellten Ausführungsform ist der Sperrfaden in Form eines Schmelzdrahtes 9 ausgeführt, der innerhalb des Elektrodenkopfes 1 und der Elektrodenzuleitung 2 mit einer Isolierung 8 versehen ist. Wird, wenn der Elektrodenkopf die vorgesehene Position erreicht hat, an die Zuleitung des Schmelzdrahtes 9 und die Wendel der Zuleitung 2 eine Spannungsquelle, bestehend aus einer Batterie 10 mit einem Schalter 12, angeschlossen und der Schalter 12 betätigt, so läßt der daraufhin fließende Strom - bei entsprechender Dimensionierung der Batterie 10 - den Draht 9 schmelzen, so daß der Fixierhaken 5 freigegeben ist.

Der spiralförmige Verlauf des Fixierhakens in seinem äußeren Bereich bewirkt in günstiger Weise ein "Festziehen" der Elektrode und damit eine Erhöhung des Anpreßdruckes im stimulierenden Oberflächenbereich derselben, wenn der Fixierhaken, nachdem er in das Gewebe eingegriffen hat durch ein weiteres Drehen der Elektrode tiefer eindringt.

In den bisher dargestellten Beispielen ist der Fixierhaken selbst federnd ausgebildet und hat das Bestreben, die Position außerhalb der Außenkontur des Elektrodenkopfes einzunehmen. Damit bildet die Verhakungsposition der Befestigungsmittel deren stabile Position, so daß diese Lage mit großer Sicherheit erreicht und eingehalten wird. Der aus einem federnden Werkstoff bestehende Fixierhaken 5 stellt eine konstruktiv besonders einfache Ausführungsform dar. In entsprechender Weise kann aber auch ein starrer Haken vorgesehen werden, der um eine feste Achse drehbar gelagert ist und beispielsweise durch eine zusätzliche Feder in die ausgeschwenkte Position geführt und dort gehalten wird. Eine schraubenförmige Ausgestaltung von Teilen des

0003948

Fixierhakens kann zwei verschiedene Aufgaben - gegebenenfalls auch gleichzeitig erfüllen:

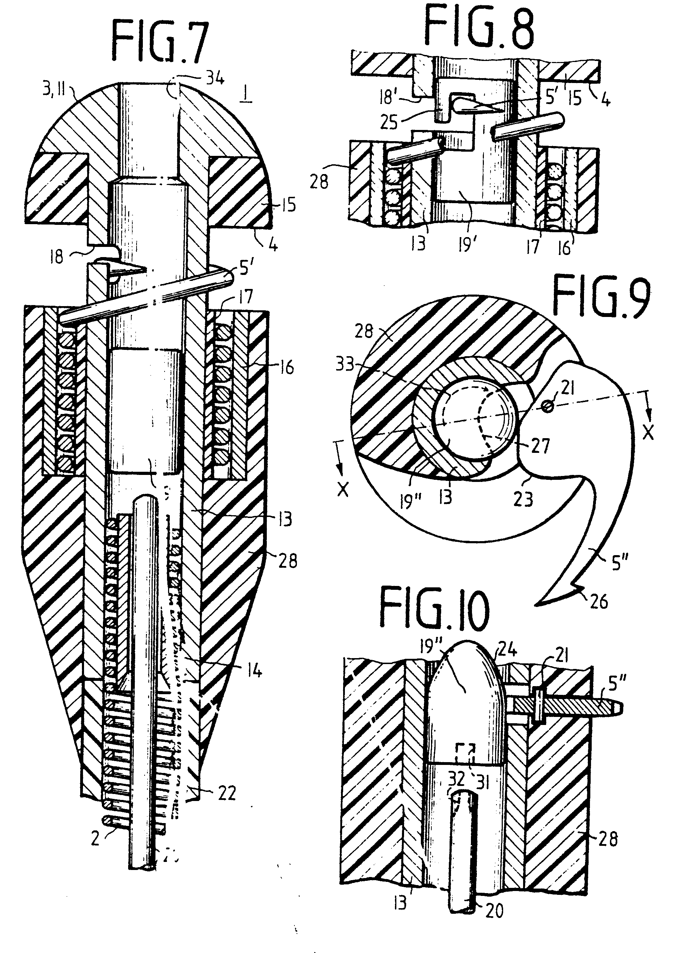

In Fig. 5 weist der äußere Teil des Fixierhakens 5 eine schraubenförmige Steigung auf. Dadurch ist es möglich, die Elektrode auch bei ausgefahrenem Fixierhaken 5 noch zurückzuziehen, falls dieses sich bei der Operation als notwendig erweisen sollte. Ist der Fixierhaken in seinem Inneren, den Grund der Ringnut umfassenden Teil mit einer schraubenförmigen Steigung gewendelt, so ergibt sich damit eine vorteilhafte Befestigungsmöglichkeit, wie es noch weiter unten anhand von Fig. 7 dargestellt werden wird.

Bei dem Ausführungsbeispiel gemäß Fig. 5 ist eine Stimulationsfläche 11 gegenüber dem übrigen Körper des Elektrodenkopfes 1 durch eine eingefügte Isolierschicht 28 isoliert, wobei die Stimulationsfläche 11 im wesentlichen mit der Frontfläche 3 des Elektrodenkopfes zusammenfällt und der Stimulationsbereich elektrisch mit der wendelförmigen Zuleitung 2 in Verbindung steht. Dadurch, daß der Stimulationsbereich von den Befestigungsmitteln elektrisch isoliert ist, lassen sich die gewünschten definierten und stabilen Potential- und Widerstandsverhältnisse erzielen, wie sie beispielsweise für die bei Herzschrittmachern vorgesehenen langandauernden Betriebszeiten erforderlich sind.

Bei der in Fig. 6 dargestellten Ausführungsvariante erstreckt sich die Stimulationsfläche seitlich bis in den Befestigungsbereich des Fixierhakens 5 hinein. Durch diese räumliche Gestaltung wird erreicht, daß der stimulierende Bereich des Elektrodenkopfes im wesentlichen mit demjenigen Bereich übereinstimmt, der an der Gewebeoberfläche anliegt. Der Fixierhaken 5 ist in dem gegenüber der Stimulationsfläche 11 durch die Isolierschicht 28 elektrisch isolierten Teil des Elektrodenkopfes (in der Zeichnung nicht sichtbar) befestigt. Durch die elektrische Trennung des Stimulationsbereiches von den Befestigungsmitteln ist es in günstiger Weise möglich, bei der Implantation der Elektrode eine Messung des Schwellenpotentials für die Stimulation vorzunehmen, ohne daß die Wirkung des Befestigungselementes, dessen Potentialverhalten im Verlaufe der Betriebszeit des Schrittmachers einen Unsicherheitsfaktor bildet, mit in die Potentialmessung eingeht. (Das das Gewebe kontaktierende Befestigungselement würde infolge des verringerten Übergangswiderstands einen zu günstigen Wert vortäuschen, der wegen der erwähnten Traumatisierung nicht über die gesamte Betriebszeit des Schrittmachers erhalten bleibt.)

Bei dem hier dargestellten Ausführungsbeispiel wird der Sperrfaden 6 statt durch eine Nut oder Bohrung durch zwei den Elektrodenkopf 1 umschlingende Bänder 29 und 30 beim Einführen der Elektrode in radialer Richtung gehalten. Die Bänder können dabei auch als Fäden oder Drähte aussgeführt sein. Die Halterung des Sperrfadens 6 mit einem umlaufenden Band bietet den Vorteil der einfachen Herstellbarkeit.

Die bisher beschriebenen Ausführungsbeispiele sind in der Zeichnung mehr oder weniger schematisch dargestellt. Bei der konstruktiven Realisierung können die Einzelteile der betreffenden Elektrodenköpfe selbst wieder aus mehreren Bestandteilen durch Verschrauben oder dergl. zusammengefügt sein.

In Fig. 7 ist ein Elektrodenkopf dargestellt, wie er sich konstruktiv besonders gut realisieren läßt. Als Trägerelement für den Elektrodenkopf 1 dient ein mit einer Kappe versehener Hohlzylinder 13, wobei die Kappe die die Frontfläche 3 bildende Stimulationsfläche 11 bildet, an die ein isolierender Formkörper 15, vorzugsweise aus Silikonkautschuk - oder einem anderen geeigneten Isolator -, anschließt. In den Hohlzylinder ist die Wendel der Zuleitung eingeschoben und mit einer inneren Klemmhülse 14 aus Metall gesichert. Die Zuleitung 2 ist wiederum mit einer Isolierung 22 ummantelt. Auf den mittleren Teil des Hohlzylinders 13 ist der schraubenförmig gewendelte Befestigungsteil des Fixierhakens 5' aufgebracht, welcher ebenfalls durch eine weitere Klemmhülse 16 aus Metall gesichert ist. Um den Fixierhaken 5' in der ausgefahrenen Position - wie bereits dargestellt - gegenüber der stimulierenden Fläche zu isolieren, ist zwischen dem wendelförmigen Teil des Fixierhakens 5' und dem Hohlzylinder 13 ein isolierendes Röhrchen 17, das beispielsweise aus polymerisiertem Tetrafluoräthylen besteht, angebracht. Der die Hülsen 14 und 16 umfassende Bereich des Elektrodenkopfes ist mit einer vulkanisierten Isolierschicht 28 (ebenfalls aus Silikonkautschuk oder einem anderen geeigneten Isoliermaterial) versehen, welche sich an die Isolierung 22 anschließt.

Die Arretierung des Fixierhakens 5' unterscheidet sich von den bisher dargestellten Ausführungen dadurch, daß er sich im eingefahrenen Zustand an einem durch eine Ausnehmung 18 gebildeten Widerlager abstützt und hier eine stabile gespannte Lage einnimmt. Um den Fixierhaken 5' freizugeben, ist ein zusätzliches in axialer Richtung bewegliches Element vorgesehen, welches aus dem isolierenden Körper

/17

19 besteht. Dieser Körper ist im Inneren des Hohlzylinders 13 beweglich und nimmt beim Einführen der Elektrode die in Fig. 7 dargestellte Position ein. Der Fixierhaken 5' erstreckt sich in seiner gespannten Stellung innerhalb der Außenkontur des Elektrodenkopfes 1 derart quer durch den Innenraum des Hohlzylinders 13, daß er von dem Körper 19 erreicht wird, wenn sich dieser im Innern des Hohlzylinders aufwärts bewegt. Damit wird nämlich der in der Ausnehmung 18 befindliche Teil des Fixierhakens 5' über das sperrende Widerlager hinweggehoben und ist damit freigegeben, so daß er in seine ausgefahrene Position zum Verhaken gelangen kann. Der Körper 19 wird seinerseits durch das Ende eines in die Elektrodenzuführung 2 eingeschobenen Führungsdrahtes 20 voranbewegt, der in erster Linie zum Versteifen der Elektrode beim Einführen in die Vene dient, und ist damit bezüglich der Kraftübertragung zwischen den Führungsdraht 20 und den Fixierhaken 5' eingeschaltet. Zum Fixieren der Elektrode ist es also nur notwendig, daß der Arzt den Führungsdraht 20 bis zum Anschlag voranschiebt, wodurch der Fixierhaken 5' freigegeben wird. Zum Erleichtern des Einführens des Führungsdrahtes 20 in die Klemmhülse 14 ist deren der Zuleitung zugewandte Öffnung trichterförmig aufgeweitet. Ist der Körper 19 aus einem für Röntgenstrahlung undurchlässigen Material gefertigt, so kann bei der Implantation mit einem Röntgengerät zusätzlich überprüft werden, ob er seine die Freigabe des Fixierhakens 5' sicherstellende Position im Elektrodenkopf erreicht hat, da sich letzterer auf dem Röntgenbild ebenfalls ausreichend deutlich abzeichnet.

Bei dieser Lösung ist besonders vorteilhaft, daß kein zusätzliches Übertragungselement notwendig ist, um vom entfernten Ende der Elektrode den Fixierhaken 5' auszulösen.

Bei der dargestellten, ein Widerlager verwendenden Arretierung ist es auch möglich, den Fixierhaken außerhalb des Körpers zunächst probeweise freizugeben und anschließend zum Gebrauch wieder in seine gespannte Position zu bringen, nachdem der zwischengeschaltete Körper 19 durch eine Bohrung 34 in der Frontfläche der Elektrode mit einem Hilfswerkzeug, welches beispielsweise eine feine Kanüle sein kann, zurückgeschoben worden ist.

Der Körper 19, der sowohl als Isoliermaterial als auch aus Metall hergestellt sein kann, übernimmt gleichzeitig die wichtige Aufgabe der Dichtung des Inneren der Elektrodenzuführung gegenüber der in die durch die Ausnehmung 18 gebildeten Öffnung eindringenden Körperflüssigkeit, welche sonst eine störende Verhärtung der Elektrodenzuführung zur Folge haben könnte. Die Verwendung eines derartigen dichtend wirkenden zwischengeschalteten Elements ist - wie ersichtlich - immer dann besonders günstig, wenn die Auslösung des Befestigngselements vom Inneren des Elektrodenkopfes her durch eine nach außen führende Öffnung hindurch erfolgen soll.

Das zwischengeschaltete Element kann - wie in Fig. 8 anhand einer Variante der Ausführungsform gemäß Fig. 7 bei einem Körper 19' dargestellt ist - auch ein schrankenförmiges Teil, d.h. hier ein Hakenteil 25, umfassen, das den Fixierhaken 5' in seiner gespannten Position im Zusammenwirken mit einer entsprechenden Gestaltung der Ausnehmung 18' bis zum Auslösen durch eine entsprechende Bewegung (in der Zeicnung nach oben) des Körpers 19' festhält.

In den Fign. 9 und 10 ist für eine Variante des in Fig. 7 dargestellten Elektrodenkopfes angegeben, wie der Fixier-

/19

haken beschaffen sein kann, wenn keine Federkräfte zur Anwendung gelangen sollen. Ein Fixierhaken 5" ist hier im wesentlichen starr ausgebildet und um eine Achse 21 drehbar gelagert (Fig. 9). Ein Ansatz 23 befindet sich im eingeklappten Zustand des Hakens 5" innerhalb des hohlen Querschnitts des Hohlzylinders 13 (gestrichelte Kontur 27). Weist ein Körper 19", der in dem Hohlzylinder 13 beweglich ist, eine entsprechend (konische) Verjüngung 24 auf (Fig. 10), so wird er, wenn er in dem Hohlzylinder nach oben gestoßen wird, den Ansatz 23 des Hakens 5" aus dem Innenbereich des Hohlzylinders 13 verdrängen und damit den Fixierhaken 5" in seine ausgeklappte Position führen. Die Linie der Berührung des Ansatzes 23 des Fixierhakens 5" mit dem Bereich der Verjüngung 24 des Körpers 19" bildet dabei eine Art Steuerkurve, welche die Bewegung des Hakens 5" in Abhängigkeit vom Voranschieben des Körpers 19" bestimmt.

Die Reibung des Körpers 19" an der Innenwand des Hohlzylinders 13 ist dabei so groß, daß er keine selbstätigen Bewegungen ausführen kann. Ist er beispielsweise durch den Führungsdraht 20 innerhalb des Zylinders in eine Endlage gestoßen worden, so ist er derart bemessen, daß er mit seiner Außenfläche ein Wiedereinklappen des Hakens 5" verhindert, der damit stabil in seiner ausgefahrenen Position gehalten ist. Der in dieser Ausführungsform verwendete Fixierhaken 5" weist an seinem Ende außerdem einen Widerhaken 26 auf, der ihn zusätzlich in seiner verhakten Position im Gewebe festhält und eine weitere Sicherung der Fixierung der Elektrode darstellt.

Das Ausklappen des Fixierhakens 5" durch Verdrängung seines Ansatzes 23 aus dem offenen Querschnitt des Hohlzylin-

/20

ders 13 durch die Außenfläche des Körpers 19" nach Art einer Steuerkurve kann auch über eine Drehung des letzteren erfolgen. Der Körper 19" weist dazu an seinem der Elektrodenzuleitung zugewandten Ende einen Schlitz 31 (in Fig. 10 gestrichelt dargestellt) und der Führungsdraht 20 einen entsprechenden Schraubenzieheransatz 32 (ebenfalls gestrichelt dargestellt) zum Eingriff in diesen Schlitz auf. Ist der Körper 19" jetzt außerdem drehbar in der Höhe des Fixierhakens 5" angeordnet und weist in seinen Querschnitt eine Ausnehmung auf, die beispielsweise der gestrichelten Kontur 27 in Fig. 9 folgt, so kann der Fixierhaken 5" zum Einführen des Elektrodenkopfes - bei entsprechender Stellung des Körpers 19" - so eingeklappt werden, daß sein Ansatz 23 in die gemäß der Kontur 27 verlaufende (nicht dargestellte) Aussparung zu liegen kommt. Wird der Körper 19" nach dem Einführen der Elektrode nun mittels des mit dem Schraubenzieheransatz 32 versehenen Führungsdrahtes 20 in Richtung des gestrichelten Pfeils 33 (Fig. 9) gedreht, so wird der Ansatz 23 durch die Oberfläche des Körpers im Bereich der Kontur 27 aus dem Innern des Hohlzylinders 13 hinausgedrückt und der Haken 5" ausgeklappt.

Es ist ersichtlich, daß bei der erfindungsgemäßen Elektrode für unterschiedliche Erfordernisse verschiedene günstige Möglichkeiten bestehen, das Befestigungselement freizugeben. So könnte beispielsweise der in Fig. 10 dargestellte Körper 19" auch seine ursprüngliche Position im Ende des vorderen Teils des Elektrodenkopfes 1 haben und mit einem Faden verbunden sein, der durch das Innere der Elektrodenzuleitung 2 hinausgeführt ist. Bei einer Verjüngung des Körpers 19" an seinem anderen Ende könnte der Fixierhaken 5" damit auch durch Ziehen an diesem Faden ausgeschwenkt werden.

Bezugszeichenliste:

Elektrodenkopf 1

Elektrodenzuleitung 2

Frontfläche 3

Ringnut 4

Fixierhaken 5, 5', 5"

Sperrfaden 6

Nut 7          .

Isolierung 8

Schmelzdraht 9

Batterie 10

Stimulationsfläche 11

Schalter 12

Hohlzylinder 13

innere Klemmhülse 14

Formkörper 15

weitere Klemmhülse 16

isolierendes Röhrchen 17

Ausnehmung 18, 18'

Körper 19, 19', 19"

Führungsdraht 20

Achse 21

schlauchförmige Isolierung 22

Ansatz 23

Verjüngung 24

Hakenteil 25

Widerhaken 26

Kontur 27

Isolierschicht 28

Bänder 29, 30

Schlitz 31

0003948

Schraubenzieheransatz 32
Pfeil 33
Bohrung 34

-.-.-.-.-

<u>P a t e n t a n s p r ü c h e</u>

1.    Implantierbare Elektrode, insbesondere zum Implantieren in einer Herzkammer zur Stimulation des Herzmuskels, mit einer isolierten Elektrodenzuleitung und mindestens einem Befestigungselement zum Fixieren des Elektrodenendes, um einen die Stimulationsfläche bildenden leitfähigen Bereich desselben mit dem Körpergewebe in Kontakt zu bringen, d a d u r c h   g e k e n n z e i c h n e t,

daß das Befestigungselement (Fixierhaken 5, 5', 5")

im Bereich des den Elektrodenkopf (1) bildenden Elektrodenendes in Axialrichtung der Elektrodenzuleitung (2) in Bezug auf dessen Frontfläche (3) zurückversetzt,

für die Einführungsphase im wesentlichen innerhalb der vom Körpergewebe tangierten Außenkontur des Elektrodenkopfes (1) unterbringbar und

zum Fixieren des Elektrodenendes derart relativ zu der Außenkontur bewegbar angeordnet ist, daß es zum Eingriff in die Gewebeoberfläche in Bezug auf die Einführungsrichtung der Elektrode hinter der Frontfläche des Elektrodenkopfes über die Außenkontur hinaus gelangt.

/2

2.. Elektrode nach Anspruch 1, dadurch gekennzeichnet, daß das Befestigungselement in im wesentlichen radialer Richtung in bezug auf die Achse der Elektrodenzuleitung (2) über die Außenkontur hinaus bewegbar angeordnet ist.

3. Elektrode nach einem der vorangehenden Ansprüche, dadurch gekennzeichnet, daß das Befestigungselement mindestens einen Fixierhaken (5, 5', 5") aufweist, der in eine diesem angepaßte Ausnehmung (Ringnut 4) des Elektrodenkopfes (1) einklappbar ist.

4. Elektrode nach Anspruch 3, dadurch gekennzeichnet, daß der Fixierhaken (5, 5', 5") im wesentlichen tangential gerichtet ist.

5. Elektrode nach einem der Ansprüche 3 oder 4, dadurch gekennzeichnet, daß der Fixierhaken (5, 5', 5") mindestens teilweise spiral- und/oder schraubenförmig ausgebildet ist.

6. Elektrode nach einem der Ansprüche 3 bis 5, dadurch gekennzeichnet, daß der Fixierhaken (5, 5', 5") im wesentlichen starr ausgebildet ist und mit seinem Eingriffsbereich aus der Außenkontur des Elektrodenkopfes (1) herausschwenkbar angeordnet ist.

7. Elektrode nach einem der vorangehenden Ansprüche, dadurch gekennzeichnet, daß das Befestigungselement mittels einer freigebbaren Sperrvorrichtung arretierbar ist.

/3

8. . Elektrode nach einem der vorangehenden Ansprüche, dadurch gekennzeichnet, daß die relative Bewegung mittels eines vom entfernten Ende der Elektrodenzuleitung (2) bedienbaren Betätigungselements (Sperrfaden 6, Führungsdraht 20) auslösbar ist.

9. Elektrode nach Anspruch 8, dadurch gekennzeichnet, daß das Betätigungselement (6, 20) in der Elektrodenzuleitung (2) geführt ist.

10. Elektrode nach einem der Ansprüche 8 oder 9, dadurch gekennzeichnet, daß das Befestigungselement mit einem beim Auslösen der relativen Bewegung wirkungsmäßig zwischengeschalteten Element (Körper 19, 19', 19") in Wechselwirkung tritt, das seinerseits durch Druck, Zug und/oder Drehung über das vom entfernten Ende der Elektrodenzuleitung (2) bedienbare Betätigungselement beeinflußbar ist.

11. Elektrode nach Anspruch 10, dadurch gekennzeichnet, daß das zwischengeschaltete Element (Körper 19, 19', 19") derart ausgebildet ist, daß es für Körperflüssigkeit dichtend an der Innenseite eines mit der die Elektrodenzuleitung (2) einerseits und dem Elektrodenkopf (1) andererseits verbundenen Hohlzylinders (13) geführt ist.

12. Elektrode nach Anspruch 11, dadurch gekennzeichnet, daß der Hohlzylinder eine seitliche Öffnung (Ausnehmung 18, 18') aufweist, durch die hindurch das zwischengeschaltete Element mit dem Befestigungselement in Wechselwirkung treten kann.

13. Elektrode nach einem der Ansprüche 10 bis 12, dadurch gekennzeichnet, daß das zwischengeschaltete Element (19, 19', 19") derart geformt ist, daß das Befestigungselement bei einer Verschiebung des Betätigungselements in axialer Richtung und/oder einer Drehung desselben durch Druck, Zug bzw. ein erzeugtes Drehmoment über die Außenkontur des Elektrodenkopfes (1) hinausbewegt wird.

14. Elektrode nach Anspruch 13, dadurch gekennzeichnet, daß die Oberfläche des zwischengeschalteten Elements (19, 19', 19") derart geformt ist, daß die Bahn des Berührungspunkts mit dem Befestigungselement eine Steuerkurve für die Bewegung des Befestigungselements bildet.

15. Elektrode nach einem der Ansprüche 3 bis 14, dadurch gekennzeichnet, daß das freie Ende des Fixierhakens (5, 5') unter der Einwirkung einer im wesentlichen radial nach außen gerichteten Federkraft steht.

16. Elektrode nach Anspruch 15, dadurch gekennzeichnet, daß der Fixierhaken (5, 5') an seinem dem Inneren des Elektrodenkopfes (1) zugewandten Ende im wesentlichen fest eingespannt ist und aus einem federnden Werkstoff besteht.

17. Elektrode nach einem der Ansprüche 7 bis 16, dadurch gekennzeichnet, daß die freigebbare Sperrvorrichtung eine aus der Bewegungsrichtung des Befestigungselements entfernbare Schranke (Sperrfaden 6) und/oder ein festes, eine Erhebung bildendes Widerlager (Ausnehmung 18, 18') aufweist, an dem das Befestigungselement bei Freigabe der
Sperrvorrichtung vorbeigeführt werden kann.

18. Elektrode nach einem der Ansprüche 7 bis 17, dadurch
gekennzeichnet, daß das Betätigungselement für die Freigabe der Sperrvorrichtung durch Zug in axialer Richtung
fadenförmig ausgebildet ist, wobei das Betätigungselement
entweder selbst eine Schranke bildet oder dem zwischengeschalteten Element in Wechselwirkung tritt, das seinerseits eine Schranke bildet oder bei Betätigung das Befestigungselement an dem Widerlager vorbeiführt.

19. Elektrode nach einem der Ansprüche 7 bis 17, dadurch
gekennzeichnet, daß das Betätigungselement für die Freigabe der Sperrvorrichtung durch Druck in axialer Richtung
in Längsrichtung hinreichend starr ausgebildet ist, wobei
das Betätigungselement entweder selbst das Befestigungselement an dem Widerlager vorbeiführt oder mit dem zwischengeschalteten Element in Wechselwirkung tritt, das
seinerseits eine Schranke bildet oder bei Betätigung das
Befestigungselement an dem Widerlager vorbeiführt.

20. Elektrode nach einem der Ansprüche 10 bis 19, dadurch
gekennzeichnet, daß das zwischengeschaltete Element derart
ausgebildet ist, daß es mit dem beim Einführen der Elektrode zur Versteifung dienenden Führungsdraht (20) derart
in Wechselwirkung treten kann, daß der Führungsdraht dabei
das Betätigungselement bildet.

/6

21. Elektrode nach einem der Ansprüche 18 bis 20, dadurch gekennzeichnet, daß das Widerlager für den unter der Einwirkung einer Federkraft stehenden Fixierhaken (5, 5', 5") durch die Form der Öffnung (Ausnehmung 18, 18') gebildet wird.

22. Elektrode nach einem der Ansprüche 18 bis 21, dadurch gekennzeichnet, daß das fadenförmige Betätigungselement (Sperrfaden 6) in axialer Richtung entlang des Elektrodenkopfes (1) geführt und im Bereich des Fixierhakens (5, 5', 5") radial gehalten ist.

23. Elektrode nach Anspruch 22, dadurch gekennzeichnet, daß das fadenförmige Betätigungselement (Sperrfaden 6) durch eine Ausnehmung (Nut 7) des Elektrodenkopfes (1) und/oder mindestens ein den Elektrodenkopf umschlingendes Band (29, 30) radial gehalten ist.

24. Elektrode nach einem der Ansprüche 10 bis 23, dadurch gekennzeichnet, daß das zwischengeschaltete Element einen Sperrhaken (Hakenteil 25) aufweist, mittels dessen der Fixierhaken (5, 5', 5") arretierbar ist.

25. Elektrode nach einem der Ansprüche 7 bis 24, dadurch gekennzeichnet, daß die Sperrvorrichtung einen zumindest einseitig isolierten Schmelzdraht (9) aufweist, der radial gehalten ist und mit einer Spannungsquelle (Batterie 10) verbindbar ist.

0003948

26. Elektrode nach einem der Ansprüche 10 bis 25, dadurch gekennzeichnet, daß das zwischengeschaltete Element (Körper 19, 19', 19") für Röntgenstrahlung undurchlässig ist.

27. Elektrode nach einem der vorangehenden Ansprüche, dadurch gekennzeichnet, daß das Befestigungelement und/oder der Elektrodenkopf mindestens einen Widerhaken (26) aufweisen.

28. Elektrode nach einem der vorangehenden Ansprüche, dadurch gekennzeichnet, daß das Befestigungselement gegenüber der Stimulationsfläche (11) des Elektrodenkopfes (1) isoliert ist.

29. Elektrode nach einem der vorhergehenden Ansprüche, dadurch gekennzeichnet, daß die Stimulationsfläche (11) im wesentlichen im Bereich der Frontfläche (3) des Elektrodenkopfes (1) und/oder im Bereich des Befestigungselements angeordnet ist.

-.-.-.-.-

FIG.1  FIG.2  FIG.3  FIG.4  FIG.5  FIG.6

0003948

— 1/2 —

FIG.7

FIG.8

FIG.9

FIG.10

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US – A – 3 835 864 (RASOR) <br> * Spalte 4, Zeilen 20-30; Spalte 5, Zeilen 28-30 und 49-51; Spalte 7, Zeilen 54-60 * | 1-8,10, 13,15- 17,28, 29 |
| X | FR – A – 2 302 107 (MEDTRONIC) <br> * Seite 4, Zeilen 12-20; Seite 7, Zeilen 28-38 und 7,8; Seite 8, Zeilen 30-39; Seite 9, Zeilen 29-32 * | 1,2,6- 10,13, 15-20, 26,28, 29 |
| | FR – A – 2 297 641 (CORDIS) <br> * Seite 3, Zeilen 1-19 und 29-30 * | 1,4,5, 27-29 |
| | DE – A – 2 133 304 (IRNICH) <br> (in der Beschreibung angeführt) <br> * Seite 3, erster Abschnitt; Seite 4, letzter Abschnitt * | 2,7-9, 11,15, 16,19 |
| | FR – A – 2 187 365 (MEDTRONIC) <br> * Seite 5, Zeilen 16-23; Seite 3, Zeilen 21-24 * | 11,27 |
| | FR – A – 1 585 065 (ALSTHOM) <br> * Seite 4, Zeilen 3-37 * | 14 |
| | US – A – 3 868 956 (ALFIDI) <br> * Spalte 1, Zeilen 48-50; Spalte 5, Zeilen 42-50; Spalte 6, Zeilen 5-10 * | 25 ./. |

**KLASSIFIKATION DER ANMELDUNG (Int.Cl.²)**

A 61 N 1/04

**RECHERCHIERTE SACHGEBIETE (Int. Cl.²)**

A 61 N 1/04
A 61 M 25/00
A 61 B 5/04
A 61 B 17/22

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 13-10-1978 | SIMON |

EPA form 1503.1  06.78

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P,E | FR - A - 2 365 351 (BENHAIM)<br>\* Seite 3, Zeilen 21-38; Figur 2 \* | 11 |

KLASSIFIKATION DER ANMELDUNG (Int.Cl.²)

RECHERCHIERTE SACHGEBIETE (Int. Cl.²)